# EUROPÄISCHE PATENTSCHRIFT

(11) **EP 0 691 043 B1**
(45) Veröffentlichungstag und Bekanntmachung des Hinweises auf die Patenterteilung: **16.07.1997**
(21) Anmeldenummer: 94911094.4
(22) Anmeldetag: 28.03.1994
(51) Int. Cl.: H01S 3/08, H01S 3/081, H01S 3/091, A61B 17/22, H01S 3/113, H01S 3/109

(54) **GÜTEGESCHALTETES LASERSYSTEM, INSBESONDERE FÜR DIE LASERLITHOTRIPSIE**
Q-SWITCHED LASER SYSTEM, IN PARTICULAR FOR LASER LITHOTRIPSY
SYSTEME LASER DECLENCHE, UTILISE NOTAMMENT EN LITHOTRIPTIE LASER

(30) Priorität: 27.03.1993 DE 4310023; 29.10.1993 DE 4336947
(43) Veröffentlichungstag der Anmeldung: 10.01.1996
(73) Patentinhaber: CLYXON LASER FÜR MEDIZINER GMBH, 12487 Berlin (DE)
(72) Erfinder: MÜLLER, Gerhard, D-14129 Berlin (DE); PASHININ, Pavel, Moscow, 117333 (RU)
(74) Vertreter: Christiansen, Henning, Dipl.-Ing.
(86) Internationale Anmeldenummer: DE9400362
(87) Internationale Veröffentlichungsnummer: WO9423478

(56) Entgegenhaltungen:
- WO-A-90/12544
- US-A- 4 525 842
- JOURNAL OF THE OPTICAL SOCIETY OF AMERICA, Bd.73, Nr.6, Juni 1983, NEW YORK US Seiten 838 - 842 M.NAKAZAWA ET AL. 'LASING CHARACTERISTICS OF A Nd3+:YAG LASER WITH A LONG OPTICAL-FIBER RESONATOR'
- JOURNAL OF THE OPTICAL SOCIETY OF AMERICA, Bd.68, Nr.11, November 1978, NEW YORK US Seiten 1621 - 1622 S.K.ISAEV ET AL. 'MODE SELF-LOCKING IN SOLID-STATE LASERS WITH LONG RESONATORS'

## Beschreibung

Die Erfindung betrifft ein gütegeschaltetes Lasersystem der im Oberbegriff des Anspruchs 1 sowie des Anspruchs 2 angegebenen Art.

Aus den Druckschriften WO 90/12544, WO 90/04358, WO 91/05332 und AT-B-380634 sind Festkörperlasersysteme für die Laserlithotripsie bekannt. Bei diesen Systemen, die mit kurzen Einzelimpulsen arbeiten, zerstört sich aufgrund der dort verwendeten kurzen Pulslängen die die Energie zum Applikationsort übertragende Faser im praktischen Gebrauch sehr schnell selbst. Darüber hinaus sind die technischen Aufbauten dieser Lasersysteme sehr kosten-und wartungsinteniv.

Überdies hat es sich bei experimentellen Untersuchungen als günstig herausgestellt, bei der Laserlithotripsie mit einem sogenannten Doppelpuls zu arbeiten, bei dem zwei Impulse kurz hintereinander zum Applikationsort übertragen werden, wobei der eine Puls zum eigentlichen energietragenden Puls zum kurzwelligen Bereich hin ("blau") verschoben ist, typischerweise die Harmonische der Grundwellenlänge darstellt. Diese Kombination erlaubt ein frühzeitiges Zünden des optischen Durchbruchs (Plasma) an der zu zerstörenden Steinoberfläche und ermöglicht damit einen höheren Wirkungsgrad bei der Steinzertrümmerung. Allerdings tritt auch hierbei der Nachteil eines schnellen Faserabbrandes im praktischen Einsatz auf.

Der Erfindung liegt daher die Aufgabe zugrunde, ein gütegeschaltetes Lasersystem der eingangs genannten Gattung anzugeben, das besonders für die Laserlithotripsie geeignete, nicht zu einem vorzeitigen Verschleiß der Übertragungsfasern führende, Laserimpulse erzeugt, effizient arbeitet und kostengünstig herstellbar ist.

Diese Aufgabe wird durch ein Lasersystem mit den Merkmalen des Anspruchs 1 gelöst.

Ausgehend von dem bekannten Stand der Technik hat sich überraschenderweise gezeigt, daß die Effizienz der Steinzertrümmerung bei der Laserlithotripsie nicht nur vom frühestmöglichen Zünden eines Plasmas und der dadurch entstehenden Stoßwelle und der Erzeugung von Kavitationsblasen abhängt, sondern entscheidend durch die Erfüllung einer Einschlußbedingung für das Plasma mit nachfolgendem Verdichtungsstoß bestimmt ist.

Theoretische und experimentelle Untersuchungen der Erfinder konnten überraschenderweise bestätigen, daß optimale Einschlußbedingungen unter Berücksichtigung der Laufzeit der akustischen Stoßwellen bei Einhaltung bestimmter Verhältnisse zwischen der Impulsdauer und der Geometrie der Endfläche der die Energie zum Applkationsort übertragenden Faser selbst gegeben sind. Als Faustregel wurde gefunden, daß die Pulsdauer für die optimale Einschlußbedingung in Nanosekunden dem 1,5fachen des Durchmessers der energieapplizierenden Fasern in Mikrometern entspricht. Das heißt also beispielsweise, bei einer Q/Q-Faser mit 360 µm Kern, die - je nach Hersteller - einen Durchmesser am optischen Cladding von etwa 400 bis 420 µm besitzt, ist die optimale Pulsdauer für die Lithotripsie ca. 600 bis 650 ns.

Die Erfindung schließt somit den Gedanken ein, das gattungsgemäße Lasersystem so auszubilden, daß es entsprechende Impulse liefert, deren Länge - je nach verwendeter Applikationsfaser - insbesondere zwischen 200 ns und 1 µs, ggf. bis zu einigen µs, liegt. Herkömmliche und aus Kostengründen zu bevorzugende laseraktive Medien wie neodymdotierte Laserkristalle, insbesondere Nd:YAG und Nd:YAIO_{3,} haben im normalen gütegeschalteten Betrieb allerdings lediglich Pulslängen zwischen 7 und 15 ns.

Erfindungsgemäß wird diese Limitierung durch eine optische Verlängerung des Resonators mittels eines langen optischen Wellenleiters überwunden. (Für eine Pulslänge von ca. 650 ns ist typischerweise eine Resonatorverlängerung mittels eines optischen Wellenleiters von ca. 18 m Länge notwendig.)

Durch diese Maßnahme wird der Effekt erzielt, daß nicht nur auf kostengünstige Weise die Pulslänge des Lasersystems in weiten Bereichen in Abhängigkeit von der Länge des Wellenleiters veränderbar ist - insbesondere zwischen 200 ns und einigen µs - , ohne daß sich die abgegebene Pulsspitzenleistung wesentlich ändert (festgestellt wurden Variationen < 10 %), sondern daß gleichzeitig eine Glättung der nachteiligen, zu erhöhtem Faserverschleiß führenden, statistischen Intensitätsoszillationen während eines Laserpulses (der sog. "Spikes") und weiterhin eine Homogenisierung des räumlichen Intensitätsprofils bei Unterdrückung von Intensitätsüberhöhungen im Strahlquerschnitt (sog. "Hot Spots") erreicht wird. Die somit bewirkte Erzeugung räumlich und zeitlich geglätteter Impulsprofile ist von erheblichem Vorteil für die Übertragung der Impulse durch angekoppelte, externe Lichtwellenleiter. Sie erhöht insbesondere deren Lebensdauer wesentlich.

Weiter schließt die Erfindung den Gedanken ein, das laseraktive Medium mit einer höheren als der zur Erzeugung von Einzelimpulsen erforderlichen Pumpenergie anzuregen, womit Impulskaskaden ("Bursts) erzeugt werden können, die - entsprechend den oben dargelegten Wirkungsbedingungen der Laserimpulsstrahlung bei der Laserlithotripsie - Wirkungsvorteile gegenüber Einzelimpulsen haben.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1a eine schematische Darstellung eines Lasersystems nach einer Ausführungsform der Erfindung,
Figur 1b eine schematische Darstellung der optischen Haupt-Komponenten eines Lasersystems nach einer gegenüber Figur 1a abgewandelten Ausführungsform der Erfindung,
Figur 2a die schematische Darstellung des Strahlenganges in einem Multipass-Wellenleiter als Bestandteil einer weiteren Ausführungsform der Erfindung und
Figur 2b eine schematische Darstellung der den MultipassWellenleiter enthaltenden Ausführungsform.

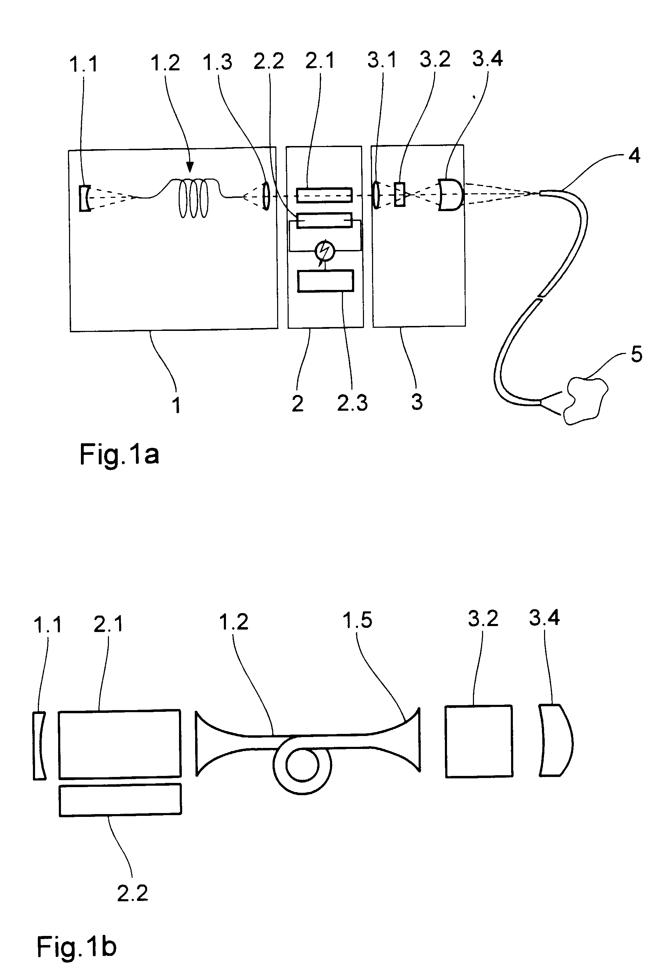

Fig. 1a zeigt schematisch eine bevorzugte optische Anordnung eines Langpuls-Festkörperlithotripsielasers als Ausführungsform der Erfindung. Hierbei besteht eine Baugruppe 1 aus einer Fokusierlinse 1.3, einer faseroptischen Resonatorverlängerung 1.2 und einem gewölbten Resonatorendspiegel 1.1, dessen Wölbung und Abstand zum Faseraustritt der Resonatorverlängerung (1.2) derart bemessen ist, daß unter Berücksichtigung der numerischen Apertur der Faser der Krümmungsradius etwa dem halben Abstand zwischen Faserendfläche und Spiegeloberfläche entspricht.

Eine Baugruppe 2 beinhaltet eine herkömmliche Laserkavität. Dabei bezeichnet 2.1 einen Laserkristall, vorzugsweise Nd:YAG oder Nd:YAl03, 2.2 eine Blitzlampe zum optischen Pumpen und 2.3 eine Einheit zur Energieversorgung und Systemsteuerung.

Eine Baugruppe 3 beinhaltet einen teiltransparenten Resonator-Endspiegel 3.4, einen nichtlinearen Kristall zur Frequenzverdopplung, typischerweise einen KTP-Kristall, sowie einen passiven Güteschalter 3.2, typischerweise Cr 4+:YAG bzw. LiF(F2-), und eine Relaisoptik 3.1 zur Kollimierung der Strahlung.

In Weiterführung des Erfindungsgedankens kann durch Einfügen einer polarisationsoptischen Baugruppe, bestehend aus einem Brewster-winkelpolarisator und einem im wesentlichen rechtwinklig hierzu angeordneten Retroreflektor, zwischen entweder den Bauelementen 1.2 und 1.3 der Baugruppe 1 oder dem nichtlinearen Kristall zur Frequenzverdopplung und dem passiven Güteschalter 3.2 der Baugruppe 3 eine weitere Steigerung der Effizienz zur Erzeugung der ersten Harmonischen erreicht werden.

Für das bevorzugte Ausführungsbeispiel lassen sich, ausgehend von einem neodymdotierten YAG-Laserkristall von 5 mm Durchmesser und 5 cm Länge, bei einer Pumpenergie an der Blitzlampe von ca. 30 J folgende typische Ausgangswerte erreichen: Pulsdauer, einstellbar zwischen 200 ns und 1 µs, abhängig von der Länge der Faserverlängerung im Resonator, bei ca. 20 m Faserlänge 160 mJ bei 1064 nm Grundemission und ca. 15 mJ bei 532 nm (2. Harmonische), ausgehend von einer Grundabsorption des passiven Güteschalters von ca. 25 %. Dabei ändert sich bei Variationen der Pulslänge von 200 ns bis 1 µs die Ausgangsenergie lediglich um ca. 10 %. Bei Verwendung von polarisierter Laserstrahlung, entweder durch Hinzufügen der oben erwähnten zusätzlichen Elemente oder durch Verwendung eines doppelbrechenden Lasermaterials, wie beispielsweise Nd:YAlO₃, erhöht sich die Ausgangsenergie der 2. Harmonischen auf ca. 22 bis 25 mJ. Dabei beträgt der Kerndurchmesser der Resonatorverlängerung lediglich 280 µm, so daß unter Berücksichtigung der durch die numerische Apertur bedingten Divergenz die Übertragung der insgesamt abgegebenen Energie durch eine Q/Q-Faser 4 mit 360 µm Kerndurchmesser zur Lithotripsie an einem Stein 5 möglich ist.

Erfindungsgemäß wird eine weitere Steigerung der Zerstörungseffizienz bei Lithotripsie dadurch erreicht, daß der Laserkristall mit einer höheren als zur Erzeugung von Einzelpulsen notwendigen Pumpenergie angeregt wird. Bereits die Erhöhung um 30 %, also auf ca. 40 J, hat die Emission einer Kaskade von Einzelimpulsen zur Folge, deren zeitlicher Abstand bei einer Grundabsorption des Güteschalters von 25 % bei ca. 50 µs liegt und deren zeitlicher Abstand bei Erhöhung der Grundabsorpion um den Faktor 2 etwa halbiert werden kann. Diese Impulskaskade (auch Burst genannt), führt zu einer wesentlichen Steigerung der Zerstörungseffizienz am Stein 5 bei der Lithotripsie, da durch die optimierten Einschlußbedingungen die Folgepulse unmittelbar zur Erzeugung von sequentiellen Verdichtungsstößen genutzt werden können.

Eine Ausführung der oben beschriebenen Anordnung ist auch als Festkörperlaser mit einem Erbium-dotierten Laserkristall, der je nach Wirtskristall bzw. Matrix Wellenlängen zwischen 1,54 und 2,94 µm emittiert. möglich. Dabei können insbesondere folgende Lasermedien zur Anwendung gelangen: Erbium:YAG, Erbium:YSGG, Erbium:YAG kodotiert mit Chrom und Thulium, sog. CTE-Laser. Als bevorzugter Wellenleiter kommt hierbei eine Zirkonfluoridfaser oder eine Aluminiumfluoridfaser zur Anwendung.

In Weiterbildung dieser Ausführung gelangen auch Erbium-Glaslaser auf der Basis von Chalkogenidgläsern (z.B. Germanium-, Arsen-, Schwefelgläsern) zur Anwendung, wobei als Wellenleiter Fasern aus dem gleichen Chalkogenidglas verwendet werden können.

Als passiver Güteschalter werden optische Kristalle, wie z.B. Lithium-Niobat bzw. Lithium-Jodat, verwendet. Es können aber auch andere als solche bekannte Güteschalter eingesetzt werden.

Auf die Verwendung von Linsen zur Strahlkonfektionierung innerhalb des Resonators kann verzichtet werden, wenn der optische Wellenleiter an beiden Enden mit einem Taper-Koppler (einer im Querschnitt in etwa keil- oder trompetenförmig zulaufenden Einkopplungsschicht) versehen ist.

Fig. 1b zeigt eine solche, gegenüber der Anordnung nach Fig. la weiterhin in der Abfolge von Laserkristall 2.1 und Wellenleiter 1.2 zwischen Spiegel 1.1 und Güteschalter 3.2 sowie teildurchlässigem Spiegel 3.4 und in der Ausbildung des Laserkristalls als Erbium-dotierter Kristall abweichende, Konfiguration mit zwei Tapern 1.5, die als konische Endstücke mit dem faseroptischen Wellenleiter 1.2 gespleißt sind.

In ähnlicher Weise können auch Holmium-dotierte Festkörperlaser sowie auch Festkörperlaser mit Frequenzvervielfachung im blauen und ultravioletten Spektralbereich und gepulste Gaslaser realisiert werden.

Es ist auch möglich, nur eines der Enden des wellenleiters mit einem Taper-Koppler zu versehen.

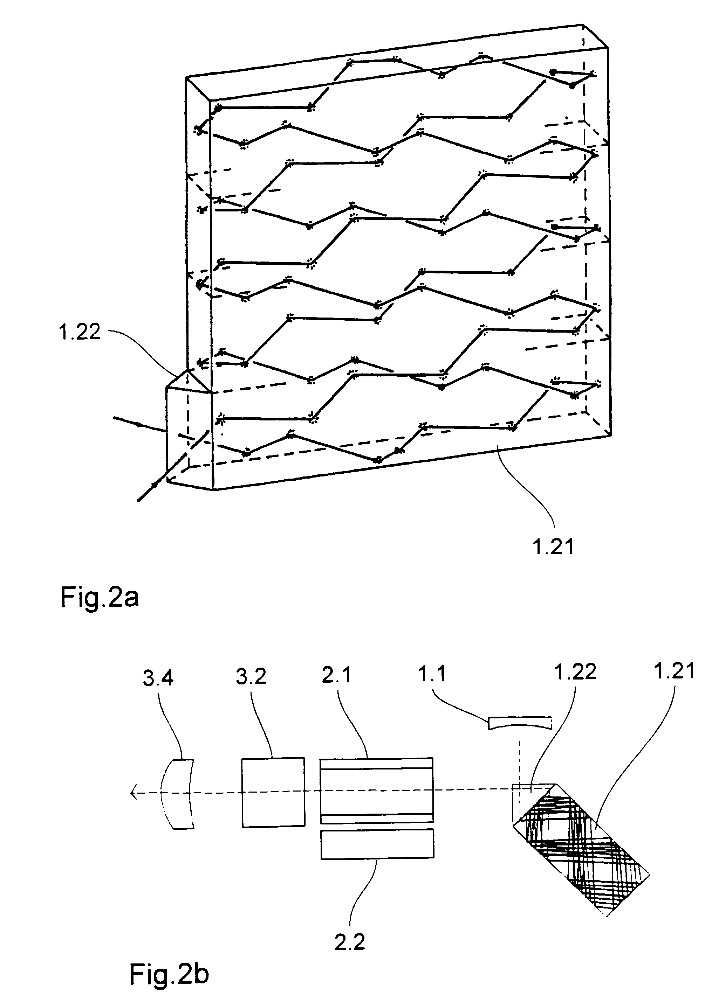

In einer anderen Ausbildung des Erfindungsgedankens kann anstelle der optischen Faser auch ein planarer optischer Wellenleiter als Multipassreflexionsplatte zur Anwendung kommen. Eine Ausführungsform eines derartigen planaren Multipasswellenleiters 1.21 ist in Fig. 2a dargestellt. Wie in Fig. 2a zu erkennen ist, hat dieser Wellenleiter im wesentlichen die Gestalt eines flachen Quaders oder einer Platte, der/die mit einem Ein-/Auskopplungsabschnitt 1.22 versehen ist und dessen große optische Weglänge durch vielfachen Strahldurchgang unter Reflexion an den Wandungen des Quaders realisiert wird.

Fig. 2b zeigt eine der in Fig. 1b dargestellten Anordnung weitgehend entsprechende Ausbildung des optischen Teils des Lasersystems unter verwendung eines Multipasswellenleiters 1.21 der in Fig. 2a gezeigten Art anstelle des in Fig. 1b dargestellten gewickelten Faser-Wellenleiters 1.2. Alle übrigen Elemente entsprechen denen der Fig. 1b und werden daher nicht nochmals erläutert.

Als Material für den planaren optischen Wellenleiter werden - je nach den Parametern der erzeugten Laserstrahlung - beispielsweise Chalkogenidgläser geeigneter Zusammensetzung (z.B. Germanium-Arsen, Schwefel) oder im entsprechenden Spektralbereich hochtransparente Kristalle, wie z.B. Saphir oder Yttrium-Aluminium-Granat oder sonstige Wirtskristalle der jeweiligen Lasermedien oder z.B. auch Silizium, Germanium oder Zinkselenid, genutzt.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

## Patentansprüche

1. Gütegeschaltetes Lasersystem, insbesondere für die Laserlithotripsie, mit einem laseraktiven Medium (2.1) in einem Resonatoraufbau, einer Anordnung zum optischen Pumpen (2.2) und einem passiven Güteschalter (3.2),
**dadurch gekennzeichnet,** daß
dem laseraktiven Medium (2.1) eine Resonatorverlängerung (1.2) zur Erhöhung der Laserimpulslänge zugeordnet ist, die einen optischen Wellenleiter aufweist.

2. Gütegeschaltetes Lasersystem, nach Anspruch 1,
**dadurch gekennzeichnet**, daß
die Anordnung zum optischen Pumpen so ausgebildet ist, daß das laseraktive Medium mit einer höheren als der zur Erzeugung von Einzelimpulsen erforderlichen Pumpenergie angeregt wird, um eine Kaskade von Impulsen (Burst) zu erzeugen.

3. Gütegeschaltetes Lasersystem nach Anspruch 1 oder 2,
**dadurch gekennzeichnet**, daß die effektive optische Länge der Resonatorverlängerung so vorbestimmt ist, daß die Ausgangsstrahlung ein zeitlich und räumlich geglättetes Impulsprofil aufweist.

4. Gütegeschaltetes Lasersystem nach einem der vorangehenden Ansprüche, **dadurch gekennzeichnet**, daß die effektive optische Länge der Resonatorverlängerung zur Erzielung einer Impulslänge im Bereich zwischen 0,2 und einigen µs wählbar ist.

5. Gütegeschaltetes Lasersystem nach einem der vorangehenden Ansprüche, **dadurch gekennzeichnet**, daß das laseraktive Medium einen Laserkristall, insbesondere aus Nd:YAG, Alexandrit oder Titansaphir oder einen solchen mit einer Er-Dotierung, aufweist.

6. Gütegeschaltetes Lasersystem nach Anspruch 5, **dadurch gekennzeichnet**, daß der Laserkristall doppelbrechende Eigenschaften aufweist, insbesondere aus Nd:YAlO₃ oder Nd:YLF besteht.

7. Gütegeschaltetes Lasersystem nach einem der Ansprüche 1 bis 4, **dadurch gekennzeichnet**, daß das laseraktive Medium gasförmig ist.

8. Gütegeschaltetes Lasersystem nach einem der vorangehenden Ansprüche, **dadurch gekennzeichnet**, daß der optische Wellenleiter eine in ihren Transmissionseigenschaften auf das laseraktive Medium abgestimmte Lichtleitfaser aus Quarzglas, Chalkogenidglas oder Zirkonfluorid aufweist.

9. Gütegeschaltetes Lasersystem nach Anspruch 8, **dadurch gekennzeichnet**, daß die Lichtleitfaser eine Q/Q-Faser mit einer numerischen Apertur von 0,2 ist.

10. Gütegeschaltetes Lasersystem nach Anspruch 8 oder 9, **dadurch gekennzeichnet**, daß die Lichtleitfaser an mindestens einem Ende mit einem angespleißten Taper versehen ist.

11. Gütegeschaltetes Lasersystem nach einem der vorangehenden Ansprüche, **dadurch gekennzeichnet**, daß der optische Wellenleiter einen gewendelten oder mäanderförmigen Aufbau zur Erreichung einer großen effektiven optischen Länge bei geringer Baulänge aufweist.

12. Gütegeschaltetes Lasersystem nach einem der vorangehenden Ansprüche, **dadurch gekennzeichnet**, daß der optische Wellenleiter einen planaren Multipasswellenleiter aufweist.

13. Gütegeschaltetes Lasersystem nach einem der Ansprüche 2 bis 12, **dadurch gekennzeichnet**, daß die Anordnung zum optischen Pumpen zur Abgabe einer um 30% oder mehr über der erforderlichen Pumpenergie liegenden Energie, insbesondere einer Energie im Bereich von 30 bis 50 J, ausgebildet ist.

14. Gütegeschaltetes Lasersystem nach einem der Ansprüche 2 bis 13, **dadurch gekennzeichnet**, daß Mittel zum Anzeigen des Auftretens von Impulskaskaden vorgesehen sind.

15. Gütegeschaltetes Lasersystem nach einem der Ansprüche 11 bis 14, **dadurch gekennzeichnet**, daß die Resonatorverlängerung als Baugruppe, bestehend aus einer abberationsarmen Kolimationslinse, einer Faserwicklung geeigneter Länge und einem konkaven Retroreflektor, aufgebaut ist.

16. Gütegeschaltetes Lasersystem nach einem der vorangehenden Ansprüche, **dadurch gekennzeichnet**, daß das System als konfokale Anordnung mit einer Kollimationslinse und einem teildurchlässigen Konkavspiegel aufgebaut ist, wobei in der Strahltaille des Systems der passive Güteschalter angeordnet ist.

17. Gütegeschaltetes Lasersystem nach Anspruch 16, **dadurch gekennzeichnet**, daß das System als Mittel zur Frequenzverdopplung einen in der Strahltaille angeordneten Verdopplerkristall aufweist.

18. Gütegeschaltetes Lasersystem nach einem der vorangehenden Ansprüche, **dadurch gekennzeichnet**, daß Mittel zum Erzeugen polarisierter Strahlung, insbesondere ein Polarisationsstrahlteiler und ein Retroreflektor, vorgesehen sind.

19. Gütegeschaltetes Lasersystem nach einem der vorangehenden Ansprüche, **dadurch gekennzeichnet**, daß das System aus drei, jeweils für sich vorjustierten und einfach montierbaren, Baugruppen aufgebaut ist.

## Claims

1. Q-switched laser system, particularly for laser lithotripsy, having a laser-active medium (2.1) in a resonator structure, an arrangement for optical pumping (2.2) and a passive Q-switch (3.2), characterised in that the laser-active medium (2.1) has an associated resonator extension (1.2) for increasing the length of the laser pulses, said extension (1.2) having an optical waveguide.

2. Q-switched laser system according to claim 1,
characterised in that the arrangement for optical pumping is constructed so that the laser-active medium is activated with a pumping energy greater than that required to generate individual pulses, so as to produce a cascade of pulses (a burst).

3. Q-switched laser system according to claim 1 or 2, characterised in that the effective optical length of the resonator extension is predetermined so that the output radiation has a pulse profile which is smoothed out in time and space.

4. Q-switched laser system according to one of the preceding claims, characterised in that the effective optical length of the resonator extension can be selected in order to achieve a pulse length in the range from 0.2 µs to a few µs.

5. Q-switched laser system according to one of the preceding claims, characterised in that the laser-active medium comprises a laser crystal, particularly of Nd:YAG, alexandrite or titanium sapphire or one which is doped with Er.

6. Q-switched laser system according to claim 5,
characterised in that the laser crystal has birefringent properties and consists particularly of Nd:YAlO₃ or Nd:YLF.

7. Q-switched laser system according to one of claims 1 to 4, characterised in that the laser-active medium is gaseous.

8. Q-switched laser system according to one of the preceding claims, characterised in that the optical waveguide has a light-conducting fibre made of quartz glass, chalcogenide glass or zirconium fluoride which is matched to the laser-active medium in its transmission properties.

9. Q-switched laser system according to claim 8,
characterised in that the light-conducting fibre is a Q/Q fibre with a numerical aperture of 0.2.

10. Q-switched laser system according to claim 8 or 9, characterised in that the light conducting fibre has a taper spliced thereto on at least one end.

11. Q-switched laser system according to one of the preceding claims, characterised in that the optical waveguide is of a coiled or meandering configuration in order to achieve a considerable effective optical length with a small construction.

12. Q-switched laser system according to one of the preceding claims, characterised in that the optical waveguide has a planar multipass waveguide.

13. Q-switched laser system according to one of claims 2 to 12, characterised in that the arrangement for optical pumping is constructed so as to deliver an energy which is 30% or more above the pumping energy required, more particularly an energy in the range from 30 to 50 J.

14. Q-switched laser system according to one of claims 2 to 13, characterised in that means are provided for indicating the occurrence of pulse cascades.

15. Q-switched laser system according to one of claims 11 to 14, characterised in that the resonator extension is constructed as an assembly consisting of a low-aberration collimator lens, a fibre coil of suitable length and a concave retroreflector.

16. Q-switched laser system according to one of the preceding claims, characterised in that the system is constructed as a confocal arrangement having a collimator lens and a partially pervious concave mirror, the passive Q-switch being located in the constriction of the beam of the system.

17. Q-switched laser system according to claim 16, characterised in that the system comprises a doubler crystal, arranged in the constriction of the beam, as the means for doubling the frequency.

18. Q-switched laser system according to one of the preceding claims, characterised in that means are provided for generating polarised radiation, particularly a polarisation beam splitter and a retroreflector.

19. Q-switched laser system according to one of the preceding claims, characterised in that the system consists of three assemblies, each individually preadjusted and easily mounted.

## Revendications

1. Système laser déclenché, notamment pour la lithotritie laser, comportant un milieu actif (2.1) dans un ensemble résonateur, un dispositif de pompage optique (2.2) et un dispositif passif de déclenchement (3.2), caractérisé en ce qu'un prolongateur de résonateur (1.2) est associé au milieu actif (2.1) en vue de l'élévation de la durée des impulsions laser, lequel présente un guide d'onde optique.

2. Système laser déclenché selon la revendication 1, caractérisé en ce que le dispositif de pompage optique est conçu de telle sorte que le milieu actif est excité par une énergie de pompage supérieure à celle requise pour la production d'impulsions isolées, pour produire une salve d'impulsions (burst).

3. Système laser déclenché selon la revendication 1 ou 2, caractérisé en ce que la longueur optique efficace du prolongateur de résonateur est prédeterminée de façon que le faisceau initial présente un profil d'impulsion temporellement et spatialement lissé.

4. Système laser déclenché selon une des revendications précédentes, caractérisé en ce que la longueur d'onde optique efficace du prolongateur de résonateur est sélectionnable en vue de l'obtention d'une durée d'impulsion de l'ordre de 0,2 à quelques µs.

5. Système laser déclenché selon une des revendications précédentes, caractérisé en ce que le milieu actif présente un cristal de laser, notamment en Nd:YAG, alexandrite ou saphir de titane, ou un cristal de laser dopé à Er.

6. Système laser déclenché selon la revendication 5, caractérisé en ce que le cristal de laser présente des propriétés biréfringentes et est notamment en Nd:YAlO₃ ou Nd:YLF.

7. Système laser déclenché selon une des revendications 1 à 4, caractérisé en ce que le milieu actif est gazeux.

8. Système laser déclenché selon une des revendications précédentes, caractérisé en ce que le guide d'onde optique présente un fibre optique en verre quartzeux, en verre aux chalcogénures ou en fluorure de zircon dont les propriétés de transmission sont adaptées au milieu actif.

9. Système laser déclenché selon la revendication 8, caractérisé en ce que la fibre optique est une fibre Q/Q ayant une ouverture numérique de 0,2.

10. Système laser déclenché selon la revendication 8 ou 9, caractérisé en ce que la fibre optique est munie à au moins une extrémité d'une transition épissée.

11. Système laser déclenché selon une des revendications précédentes, caractérisé en ce que le guide d'onde optique présente une configuration en spirale ou en méandre pour atteindre une grande longueur optique efficace pour un encombrement faible.

12. Système laser déclenché selon une des revendications précédentes, caractérisé en ce que le guide d'onde optique présente un guide d'onde optique plan à trajets multiples.

13. Système laser déclenché selon une des revendications 2 à 12, caractérisé en ce que le dispositif de pompage optique est conçu pour céder une énergie supérieure de 30 % ou plus à l'énergie de pompage requise, notamment une énergie de l'ordre de 30 à 50 J.

14. Système laser déclenché selon une des revendications 2 à 13, caractérisé en ce que sont prévus des moyens indiquant la survenue de salves d'impulsions.

15. Système laser déclenché selon une des revendications 11 à 14, caractérisé en ce que le prolongateur de résonateur est conçu en tant qu'ensemble composé d'une lentille de collimation à faible aberration, d'un rouleau de fibre de longueur appropriée et d'un réflecteur concave.

16. Système laser déclenché selon une des revendications précédentes, caractérisé en ce que le système est constitué en tant que dispositif confocal comportant une lentille de collimation et un miroir concave partiellement transparent, le dispositif passif de déclenchement étant disposé dans la partie resserrée du faisceau du système.

17. Système laser déclenché selon la revendication 16, caractérisé en ce qu'il présente un cristal doubleur disposé dans le trajet du faisceau en tant que moyen de doublement de la fréquence.

18. Système laser déclenché selon une des revendications précédentes, caractérisé en ce que sont prévus des moyens de production d'un faisceau polarisé, notamment une lame séparatrice du faisceau de polarisation et un réflecteur.

19. Système laser déclenché selon une des revendications précédentes, caractérisé en ce que le système est constitué de trois ensembles individuellement pré-ajustés et de montage simple.
